# EUROPEAN PATENT APPLICATION

(11) **EP 4 239 314 A1**
(43) Date of publication of application: **06.09.2023**
(21) Application number: 22460069.2
(22) Date of filing: 15.12.2022
(51) Int. Cl.: G01N 17/02, G01N 17/04

(54) **METHOD OF APPLICATION OF A SENSOR FOR CORROSION RATE OF REINFORCED CONCRETE STRUCTURES**

(30) Priority: 31.01.2022 PL 44027722
(71) Applicant: Politechnika Slaska, 44-100 Gliwice (PL)
(72) Inventor: Jasniok, Tomasz, 43-190 Mikolów (PL)

(57) **Abstract**

1. A method of application of a sensor for corrosion rate of reinforcement, particularly in existing reinforced concrete structures **characterized in that** a sensor comprising a working electrode **6**, a reference electrode **7**, and a counter electrode **8** in the form of a C-shaped strip are placed in the plastic housing **5** and covered with resin **9**, followed by smoothing the common surface of a sensor **10**, electrodes **6**, **7**,and **8**, and resin **9**, exposed to external factors, to the roughness class not greater than Ra 0.63, then the bottom **3** of an opening **2** in the concrete member **1** is smoothed to the roughness class not greater than Ra 1.25, then mechanical pressure of smooth flat surfaces of concrete **3** and the electrodes **6**, **7**, and **8** in the sensor providing charge transfer through concrete between the electrodes maintains stress until resin **11** stabilising the corrosion sensor **4** in the opening **2** is cured, followed by measuring polarization with the electrochemical methods.

## Description

The present invention provides the method of application of a sensor for corrosion rate of reinforcement, particularly in existing reinforced concrete structures.

Reinforced concrete structures are exposed to aggressive environment, which causes their destruction due to, inter alia, electrochemical corrosion of reinforcement. It is very difficult to recognise and identify corrosion processes because they take place under concrete cover. Only when the cover cracks due to accumulated corrosion products, progression of reinforcement corrosion can be identified directly. But cracks in concrete indicate already very advanced corrosion and necessity to repair the structure. Therefore, to identify reinforcement corrosion prior to concrete destruction a decision is made to conduct diagnostic tests. According to *Zybura A., Jaśniok M., Jaśniok T.: Diagnostyka konstrukcji zelbetowych. T. 2. Badania korozji zbrojenia i w* 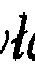 *aściwości ochronnych betonu, Wydawnictwo Naukowe PWN, Warszawa, 2011* for more than 30 years the methods employed in electrochemical laboratories, which use a three-electrode system, in which steel reinforcement of a reinforced concrete member is used as a working electrode, and additionally a reference electrode with constant and known potential in the form of a head and a corrosion-resistant auxiliary electrode are applied to concrete surface, have been adopted to reinforced concrete structures. The whole system is connected to a potentiostat and a measurement is taken with one of the electrochemical methods: Linear Polarization Resistance (LPR), Electrochemical Impedance Spectroscopy (EIS), or Galvanostatic Pulse (GP).

Values of density of corrosion current, which according to Faraday's law clearly defines the current rate of reinforcement corrosion (specifies the decrement of rebar mass over time).

Tests may be performed using a head present on the existing reinforced concrete structure, but they may be burdened with an error, a multi fold one, related to an unknown analysed surface of reinforcement (the polarized area), whose value is substantial for determining a correct value of corrosion current density - Andrade C., Alonso C.: On-site measurements of corrosion rate of reinforcements, Construction and Building Materials, 2001, vol. 15. pp. 141-145*.*

The solution may be an application of heads with the so-called guard ring, which is described by Feliu S. et al.: Possibilities of the guard ring for electrical signal confinement in the polarization measurements of reinforcements, Corrosion, 1990, vol. 46. pp. 1015-1020*),* however measuring errors related to underestimated polarized area may occur in this case.

The method of limiting and identifying the polarized area of a rebar during polarization measurements of corrosion rate of reinforcement, particularly in reinforced concrete structures, in which a cut in concrete cover filled with resin substantially limits the polarization range, is known from patent description PL236339, but similarly as in case of the method with the "guard ring", the polarized area may be underestimated in certain conditions.

The solution for a problem of an unknown polarization range in tests on corrosion rate of reinforcement in concrete is the application of sensors with the independent three-electrode system described e.g. by Dong S. G. et al.: Effective monitoring of corrosion in reinforcing steel in concrete constructions by a multifunctional sensor, Electrochimica Acta, 2011, vol. 56. pp. 1881-1888 or Yu H., Caseres L.: An embedded multi-parameter corrosion sensor for reinforced concrete structures, Materials and Corrosion, 2012, vol. 63. pp. 1011-1016*.* The working electrode has a limited size and is polarized in whole, thus the analysed area is known. Unfortunately, the application of the sensor for testing corrosion rate of reinforcement is possible only in newly-built structures, and the sensor may be placed in formwork before placing the concrete.

When an effort is made to apply such sensors in existing structures, it is necessary to adhere the sensor into a previously prepared opening, however a layer of binder between concrete and the working electrode of a sensor affects factors which induce corrosion because this layer acts as a new barrier separating aggressive concrete from the electrode. Therefore, the obtained results for corrosion rate of reinforcement would correspond to conditions in the binder, and not in aggressive concrete.

The solution for this problem is presented in the patent description PL235314, in which the contact between aggressive concrete and the working electrode (reinforcement) is maintained by their jointly cut out as core from a concrete member, and after fixing the auxiliary electrode and the reference electrode in the core, the core (not only the electrodes) is adhered into an opening left after the previously cut-out core. The disadvantage of the above solution is its labour and time consumption because the cut-out core has to be transported to a laboratory, where it is "equipped" with additional electrodes and again transported back to the facility to be adhered there.

Development of a new, innovative method of production and application of a sensor for the existing reinforced concrete structures to eliminate the current inconveniences known from the state art, is a technical problem to be solved.

The method of application of the sensor for corrosion rate of reinforcement, particularly in the existing reinforced concrete structures consists in the fact that the sensor comprising the working electrode, the reference electrode, and the counter electrode in the form of a C-shaped strip is placed in a plastic housing and covered with resin, then the common surface of the sensor for electrodes and resin, exposed to external factors, is smoothed to the roughness class not greater than Ra 0.63, followed by smoothing the opening bottom in the concrete member to the roughness class not greater than Ra 1.25, and then stress to set resin, which stabilises the corrosion sensor in the opening, is maintained by mechanically pressing down smooth flat surfaces of concrete and electrodes in the sensors to provide transfer of the charge through concrete between the electrodes, which is followed by measuring polarization with electrochemical methods.

Preferably, in the method of sensor application according to the present invention, the working electrode is made of reinforcing steel.

Preferably, in the method of sensor application according to the present invention, the counter electrode is made of metal resistant to electrochemical corrosion.

Preferably, in the method of sensor application according to the invention, electrodes in the sensor are electrically insulated from one another by the infill in the form of epoxy resin.

Preferably, in the method of sensor application according to the present invention, epoxy or polyester resin is used as resin for stabilising the sensor in the opening.

Preferably, in the method of sensor application according to the present invention, the method of: linear polarization resistance (LPR), electrochemical impedance spectroscopy (EIS), or galvanostatic pulse (GP) is used as the electrochemical polarization method.

The advantage of this solution according to the present invention is that single measurements may be performed or corrosion rate may be continuously monitored with electrochemical methods for a known surface of the working electrode, additionally the advantage is the application of the sensor in concrete in the existing structure without using a layer of binder between the electrodes in the sensor and concrete. The solution allows for obtaining from tests values of corrosion rate typical for the current aggressiveness of concrete in the structure, and not for the binder layer.

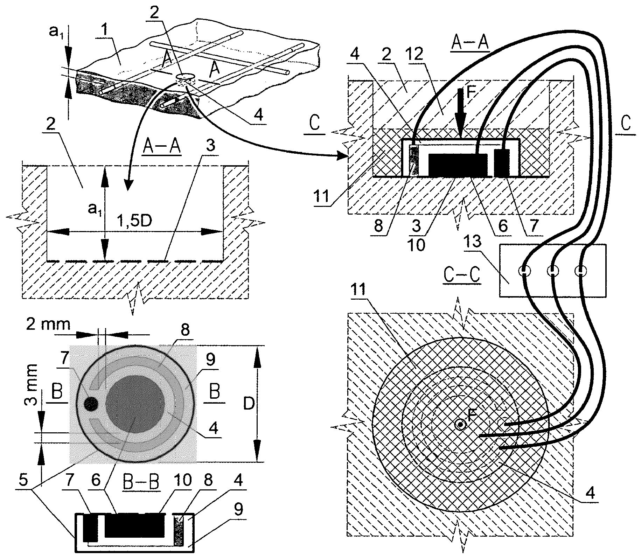

The object of the invention in the embodiment is illustrated in the drawing as a diagram of the method of sensor application in the slab element of the reinforced concrete structure.

### Example

In a reinforced concrete slab member **1** an opening **2** was performed having a depth equal to the depth of concrete cover ai in accordance with standard PN-EN 1992-1-1:2008 and a diameter of 1.5D, whose bottom comprising concrete surface **3** was smoothed to roughness Ra 1.25 in accordance with standard PN-EN ISO 1302:2004. A working electrode **6** in the form of a roller made of reinforcing steel grade B500SP, a reference electrode **7** in the form of a graphite roller within a distance of 2 mm from the working electrode **6** and an auxiliary electrode **8** in the form of a C-shaped strip prepared from conductor made of steel grade X5CrNi18-10 resistant to electrochemical corrosion and within a distance of 3 mm from the working electrode **6,** were placed in the corrosion sensor **4,** whose housing **5** with a diameter D is made of plastic,. Electrical insulation of all elements of the sensor was provided by filling the housing **5** with epoxy resin **9,** whose surface **10** common with the electrodes **6, 7** and **8** was smoothed to roughness Ra 0.63 in accordance with standard PN-EN ISO 1302:2004, followed by smoothing the bottom **3** of the opening **2** previously made in the concrete member **1** to the roughness class not greater than Ra 1.25 in accordance with standard PN-EN ISO 1302:2004. The sensor surface **10** was applied to the smoothed concrete **3** and mechanically pressed down with force **F** that caused stress in the contact area between concrete **3** and the sensor surface **10** equal to a half of the average tensile strength of concrete in accordance with standard PN-EN 1992-1-1:2008. The force **F** was exerted until achieving the final strength of resin **11** which filled space between concrete in the opening **2** and the corrosion sensor **4.** The opening **2** was closed and smoothed with patching mixture **12.** Connecting the electrodes **6, 7,** and **8** and the corrosion sensor **4** to a potentiostat **13** allowed for conducting electrochemical tests with the method of Linear Polarization Resistance (LPR), Electrochemical Impedance Spectroscopy (EIS), or Galvanostatic Pulse (GP).

## Claims

1. A method of application of a sensor for corrosion rate of reinforcement, particularly in existing reinforced concrete structures **characterized in that** a sensor comprising a working electrode **6,** a reference electrode **7,** and a counter electrode **8** in the form of a C-shaped strip are placed in the plastic housing **5** and covered with resin **9,** followed by smoothing the common surface of a sensor **10,** the electrodes **6, 7**, and **8,** and resin **9,** exposed to external factors, to the roughness class not greater than Ra 0.63, then the bottom **3** of an opening **2** in the concrete member **1** is smoothed to the roughness class not greater than Ra 1.25, then mechanical pressure of smooth flat surfaces of concrete **3** and the electrodes **6**, **7**, and **8** in the sensor providing charge transfer through concrete between the electrodes maintains stress until resin **11** stabilising the corrosion sensor **4** in the opening **2** is cured, followed by measuring polarization with the electrochemical methods.

2. A method of application of the sensor, according to claim 1, **characterized in that** the working electrode **6** is made of reinforcing steel.

3. A method of application of the sensor according to claim 1, **characterized in that** the counter electrode **8** is made of metal resistant to electrochemical corrosion.

4. A method of application of the sensor for corrosion rate of reinforcement **characterized in that** electrodes **6, 7,** and **8** in the sensor are electrically insulated from one another by the infill 9 in the form of epoxy resin.

5. A method of application of the sensor for corrosion rate of reinforcement **characterized in that** epoxy or polyester resin is used as resin **11** for stabilising the sensor in the opening.

6. A method of application of the sensor, according to claim 1, **characterized in that** the method of linear polarization resistance (LPR), electrochemical impedance spectroscopy (EIS), or galvanostatic pulse (GP) is used as the electrochemical polarization method.
